# EUROPEAN PATENT SPECIFICATION

(11) **EP 3 866 672 B1**
(45) Date of publication and mention of the grant of the patent: **29.05.2024**
(21) Application number: 19805734.1
(22) Date of filing: 14.10.2019
(51) Int. Cl.: A61B 5/00, A61B 5/053

(54) **WEARABLE MEASURING DEVICE THAT CAN BE WORN ON A PERSON S BODY**
WEARABLE-MESSVORRICHTUNG, DIE AM KÖRPER EINER PERSON GETRAGEN WERDEN KANN
DISPOSITIF DE MESURE PORTABLE POUVANT ÊTRE PORTÉ SUR LE CORPS D'UNE PERSONNE

(30) Priority: 17.10.2018 BE 201805713
(43) Date of publication of application: 25.08.2021
(73) Proprietor: Monoa BVBA, 9200 Dendermonde (BE)
(72) Inventor: DE VOS, Renaat, 9940 Evergem (BE); BOGAERTS, Stijn, 9200 Dendermonde (BE)
(74) Representative: Van hunsel, Lieven M.S.
(86) International application number: PCT/IB2019/058730
(87) International publication number: WO 2020/079559

(56) References cited:
- WO-A1-2017/165532
- WO-A2-2014/205434
- WO-A2-2015/101947
- US-A1- 2004 039 254
- US-A1- 2014 275 888
- US-A1- 2016 058 366
- US-A1- 2016 317 060
- US-A1- 2018 143 663
- US-A1- 2018 288 586

## Description

The present invention concerns a wearable measuring device that can be worn on a person's body.

The common English terminology in the technical field to which the invention relates includes terms such as 'wearable', 'wearable technology', 'wearable devices', which describe a variety of devices or gadgets that a person wears as a garment or accessory on his or her body and which contain technology to measure, collect and, if necessary, transmit data on the body's condition to other devices.

In French, there is also mention of the so-called "habitronique", which is a composition of "s'habiller" (meaning "to dress") and "electronique".

In short, a wearable measuring device according to the invention can be considered as an element that belongs to the technical domain of the aforementioned electronic devices that can be worn by a person on the body.

More specifically, the invention relates to such a wearable measuring device that is intended to measure parameters on the person's skin.

Such parameters can take many forms, but according to the invention will typically (but not necessarily) relate to physical parameters related to the activity of the sweat glands in the skin, such as, for example, the electrical conductivity of the skin, the speed or magnitude at which this conductivity of the skin changes and so on.

Indeed, a change in the activity of the sweat glands reflects a change in the intensity of the emotional state or excitement of the person concerned.

The aim is that a wearable measuring device according to the invention can monitor a person's emotional state, state of mind or excitement and that the data collected by the measuring device can be offered to the person, typically by means of a separate output device, such as an app on a mobile phone or on any communication device, or by means of software installed on a PC, laptop or tablet or on any other device that is appropriate.

According to the present state of the art, many electronic devices are known that can be worn on the body by a person (the so-called "wearables"), but a number of needs cannot be met at present with these known devices.

For example, there are the well-known watches with built-in technology (the so-called "smartwatches") for monitoring, for example, sports performances, the heart rate, and so on.

In other words, in this "intelligent" type of watches, the original functions relating to the display of date and time and so on, together with the functions relating to the measurement of data on the person's skin and the storage and monitoring of these data, are integrated into a single device, in this example the watch.

This integration of functions may have advantages, but it also has disadvantages.

More specifically, the makers of technology for measuring data on the skin are not primarily manufacturers of watches and vice versa.

Consequently, the watches with integrated measurement technology in most cases look rather futuristic with a very digital look.

This is mostly not annoying when it comes to sports performances, for example, but when the measurement technology is used for continuous monitoring of the individual's state of mind, it is often undesirable to have to wear a flashy electronic device around the wrist all day long.

This is particularly the case, for example, for persons who are expected to wear more formal clothing at work, such as office workers, salesmen, lawyers, judges and the like.

Another disadvantage of the aforementioned integration of functions is that when buying a watch, there are many models in which the measurement technology is not integrated and only a few models in which this measurement technology is integrated, so that the choice when buying a watch is very limited if, in addition to the time display, one is also looking for technology that is able to measure the emotional state.

Other similar electronic devices or 'wearables' can be glued to the body or may have the shape of a flexible bracelet or the like.

A disadvantage of these electronic devices or "wearables" is again that they are usually very flashy, which is often undesirable for the aforementioned reasons.

Furthermore, they are generally not very durable or robust and are intended rather as a gadget or novelty for temporary experimentation, or for short-term use.

Another disadvantage of these embodiments is that they cover a fairly large part of the skin surface, which can lead to itching, irritation and even infections.

US2016058366 discloses wearable electronic devices, systems, and methods for monitoring sleep. Motion sensor values of the current motion of the electronic device are acquired and the change in motion intensity of an electronic device is calculated by comparing motion sensor values over two or more time periods. If the change in motion intensity fits a predetermined pattern, it is determined whether the electronic device is currently being worn. If it is determined that the electronic device is currently being worn, sleep monitoring is performed.

The electronic device includes a main body (functional device portion) and a wearing part (including a band or a strap) including wearing members. The main body may be configured to be capable of being attached to or removed from the wearing part. The main body has a substantially rectangular bar-type shape which at least partially has a curvature corresponding a user's body. The main body comprises a sensor module which might include a galvanic skin response sensor.

WO2015126179 teaches a portable device which may include a strap including a substantially polygonal opening and a loop formed at one end thereof; a buckle portion configured to secure opposite ends of the strap to each other by connecting opposite ends of the strap; and a body configured to be coupled to the opening by being fitted into the opening or decoupled from the opening. The body may include a front surface and a rear surface, and a display device having a substantially polygonal shape when viewed from above on the front surface, and a biometric sensor disposed on the rear surface.

WO2017165532 discloses a biosensor module for detecting one or more biosignals at a ventral wrist of a user is disclosed. The module includes a housing having an inner surface facing the wrist and an outer surface not facing the wrist, both formed of an insulating material, accommodating one or more processing units between the inner and outer surfaces ; and one or more biosensors protruding from the inner wrist facing surface and electronically coupled to the processing unit or units within the housing. The invention also relates to an assembly, comprising a support member comprising a portion adapted to receive a wrist band; and a biosensor module comprising a housing having an internal surface facing the wrist and an external surface not facing the wrist, the housing being curved in the direction facing the wrist and accommodating one or more processing units between the internal surfaces and external, and one or more biosensors protruding from the internal surface facing the wrist and electronically coupled to the processing unit.

US2004039254 teaches an apparatus for detecting human physiological or contextual information which includes flexible sections, a housing removably attached thereto, one or more sensors and a processor. The apparatus may include one of several structures for removably attaching the housing to the flexible sections and may have adjustable operating parameters. An alternate apparatus measures heat flux and includes a housing, a known resistivity base member, a processing unit and two temperature measuring devices attached to the base member, one in thermal communication with the body through a thermal energy communicator and the other in thermal communication with the ambient environment. A further alternate apparatus includes a housing having an adhesive material on a surface thereof for removably attaching the housing to the body. A further alternate apparatus includes a housing having an inner surface having a concave shape in a first direction and convex shape in a second direction substantially perpendicular thereto. An apparatus for detecting heart related parameters of the wearer includes an acoustic-based non-ECG heart parameter sensor and one or more filtering sensors for generating a signal related to the non-heart related motion of the body, the second signal being used to subtract an acoustic component generated from non-heart related motion of the body from the non-ECG heart parameter sensor's signal to generate a third signal used to generate the heart related parameters of the wearer.

None of the existing apparatus are suitable for solving the problems as discussed in the present patent application with respect to accuracy, efficiency, comfort, technical and economic viability.

The current invention therefore aims to provide a solution to the aforementioned problem and/or other problems.

In particular, the aim of the invention is to provide a wearable measuring device that is very durable and robust, so that it will have a long service life and can withstand shocks or forces that are commonly exerted by a person in motion, as well as any penetration of moisture into the measuring device.

Another aim of the invention is to provide the technology used to measure a person's skin in one separate wearable measuring device, which preferably has a very small volume, so that it can be worn discreetly.

Another aim of the invention is to supply a measuring device that a user can wear unnoticed or almost imperceptibly on, in or under his daily clothing or other accessories worn by the user, such as watches, bras or belts, in such a way that the user does not have to change his vestimentary habits and style in any way, and can continue to purchase clothing and accessories from the ordinary wide range of products available on the market, as in the past.

Yet another aim of the invention may be to offer a wearable measuring device that is not only durable, but also exudes a certain standing or is at least designed such that it can also be worn by persons who attach great importance to class and style.

On the other hand, in another possible embodiment, one possible aim of the invention is to provide a durable wearable measuring device with an emphasis on its wide distribution, for example by trying to keep its cost price as low as possible.

To this end, the present invention concerns a wearable measuring device that can be worn on a person's body, intended to measure parameters on the person's skin, carried out like a measuring block fitted with an elongated, slightly curved housing, more specifically as if it were a segment of a cylindrical sheath with a radius between 52 and 62 millimetres, or rather between 54 and 60 millimetres, and preferably between 56 and 58 millimetres, with a convex side and a concave side, and having in the surface of the concave side at least two electronic sensors intended to be put into contact with the skin on the inside of the wrist of the person concerned to measure the parameters, wherein the wearable measuring device is not equipped with a wristband or the like,
whereby the wearable measuring block is suitable to be located between a wristband, for example of a usual wristwatch, and wrist, whereby the measuring block is suitable to be used between the wristband and the skin on the inside of the wrist and is provided to this end with fixing means, characterised in that the fixing means contain a hook or clips which can be hooked behind or around a part of a said wristband, whereby the measuring block (2) contains at least two electronic sensors (9, 10) for measuring the activity of the sweat glands in the area concerned of the skin (11), also known as 'galvanic skin response', wherein at least two sensors are provided at a mutual distance of at least 20 millimetres and wherein the sensors have a rounded contact surface.

A major advantage of such a wearable measuring device according to the invention is that it has a housing whose concave side can be fitted over a convex part of the skin at the wrist so that a good contact between the skin and the sensors in the wearable measuring device is ensured.

Another advantage of such a wearable measuring device according to the invention is that it is designed as a separate measuring block with a housing in which the electronic sensors are provided.

In this way, a robust and compact version can be realized on the one hand, and the measuring technology is contained in a separate block on the other hand, so that such a measuring block in a particular embodiment can be combined with many types of watches and other wristbands, for example.

According to the invention, the measuring block contains at least a pair of electronic sensors for measuring the activity of the sweat glands in the skin zone concerned, also called `galvanic skin response', wherein at least two sensors are provided at a mutual distance of at least 20 millimetres and wherein the sensors have a rounded contact surface.

If the distance is long enough, a battery can be fitted between the sensors.

A major advantage of the invention is that it allows to detect and record the state of mind or more physical parameters such as the heartbeat of the wearer, so that this knowledge can be used at a later stage or simultaneously in all kinds of apps or software programs, for example to improve the general well-being of the person concerned or monitor or preventively adjust his or her general condition.

According to the invention, the wearable measuring block is designed to be attached to a wristband, for example of a usual wristwatch, in particular to be used between the wristband and the skin on the inside of the wrist and it is provided with fixing means to this end.

A major advantage of such a wearable measuring device according to the invention is that the garment or accessory such as a watch to which it is attached ensure a good contact with the user's skin.

The wearable measuring device is adapted to the watch or wristband, but any embodiment of the intended type can serve to attach such a wearable measuring device to it, regardless of the style or detailed shape or colour it has.

This increases the choice of style and shape for a user enormously.

Because the wearable measuring device is attached to a wristband, possibly of a conventional watch, it does not have its own means of applying it to the user's body, which makes it relatively small in size and allows it to be discreetly applied to the body for measuring the relevant parameters.

In another preferred embodiment of a wearable measuring device according to the invention, the wearable measuring device is equipped with transmitting devices to wirelessly transmit data to an output device, such as a mobile phone, a communication device in general, a PC, laptop, iPod, mp3 player or the like.

The invention also concerns a combination of a wearable measuring device according to the invention and an output device, wherein the output device is equipped with means for receiving data from the measuring device wirelessly and with an app or software application capable of processing said data and of presenting it to a user in a processed form.

In order to better explain the characteristics of the invention, some preferred embodiments of a wearable measuring device according to the invention are described below as an example only without being limitative in any way, with reference to the attached figures in which:
figure 1 shows a view in perspective of the convex side of a first possible embodiment of a wearable measuring device according to the invention;
figure 2 shows a view in perspective according to arrow F2 on the concave side of the wearable measuring device in figure 1;
figures 3, 4 and 5 show a top view, a first side view and a second side view respectively according to arrows F3, F4 and F5 on the wearable measuring device from figure 1;
figure 6 shows a cross-section through the wearable measuring device from figure 1 according to the section VI-VI indicated in figure 3;
figure 7 shows a disassembled version of the wearable measuring device from figure 1 seen in perspective;
figure 8 illustrates the use of the wearable measuring device from figure 1 seen in side view;
figures 9 to 13 included show another possible embodiment of a wearable measuring device according to the invention in a similar way as in figures 1 to 5 included;
figure 14 illustrates the use of a wearable measuring device in the embodiment according to figure 9 seen in side view;
figures 15 to 19 included show a third possible embodiment of a wearable measuring device according to the invention in a way similar to that shown in figures 1 to 5 included;
figures 20 to 24 included show a fourth possible embodiment of a wearable measuring device according to the invention in a manner similar to that shown in figures 1 to 5 included;
figure 25 illustrates the use of a wearable measuring device in the embodiment according to figure 20 seen in side view;
figure 26 illustrates the use of a wearable measuring device in a slightly modified embodiment compared to the embodiment of figures 20 to 25 included, seen in side view; and
figure 27 illustrates the charging of a wearable measuring device according to the invention in an embodiment as shown in figure 1, seen in side view.

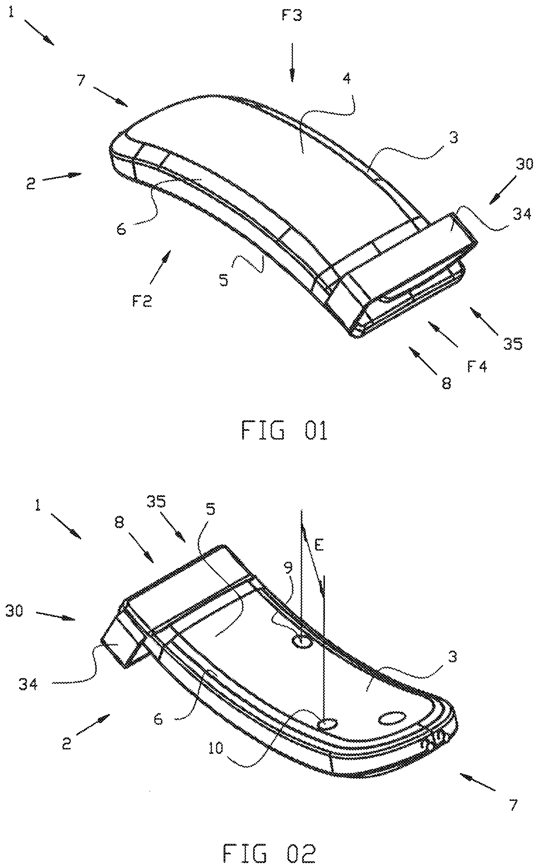

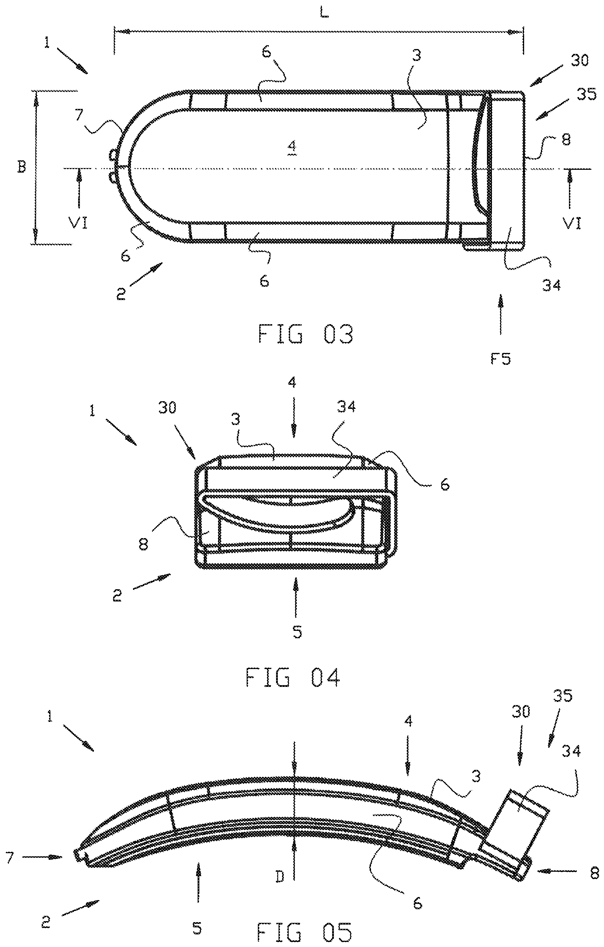

The wearable measuring device 1 according to the invention shown in figures 1 to 7 included is intended to measure parameters on a person's skin.

The wearable measuring device is designed as a closed unit in the shape of a measuring block 2 that is equipped with an elongated, shell-shaped housing 3.

This housing has a convex side 4 and a concave side 5.

In addition, the shell-shaped housing 3 has a constant or nearly constant width B and a constant or nearly constant thickness D over the greater part of its length L.

For example, the length L may typically be between 50 and 60 mm, but the invention does not rule out the possibility of using a different length L.

The width B corresponds to the width of a watch strap and may, for example, be in the order of magnitude of 16 mm to 24 mm, but other dimensions are not excluded from the invention.

The thickness D is preferably as thin as possible so that the wearable measuring device can be discretely fitted between a watch strap worn by the user and the user's skin.

For example, the thickness D may typically be 6 to 7 mm over the greater part of the surface of the wearable measuring device 1. Of course, thinner as well as thicker versions are not excluded from the invention.

At the edges 6, the wearable measuring device 1 in this example is slightly rounded or chamfered and the thickness D slightly decreases, for example to about 4 to 5 mm.

Of course, these values are merely possible examples and, according to the invention, it is not excluded to use completely different shapes and sizes.

In the embodiment shown in Figures 1 to 7, the wearable measuring device 1 is provided with a front side 7 that is semi-circular or almost semi-circular and a back side 8 that in this case is rectilinear or almost rectilinear, but according to the invention this does not have to be the case.

In the surface of the concave side 5, according to the invention, at least a pair of electronic sensors 9 and 10 are provided, which are intended to be brought into contact with the user's skin 11 for measuring the aforementioned parameters.

According to the invention, this pair of electronic sensors 9 and 10 is provided in the measuring block 2 typically intended to measure the activity of the sweat glands in the relevant part of the skin 11.

In order to know this activity, the so-called 'galvanic skin response' can be measured, wherein parameters such as, for example, the electrical conductivity on the skin 11 or changes in this electrical conductivity are measured.

However, the invention does not rule out the possibility of measuring other parameters additionally or alternatively, by providing sensors of a different nature or by increasing or decreasing the number of sensors.

The electronic sensors 9 and 10 should preferably be situated at a sufficient intermediate distance E, for example at a distance E of approximately 2 cm, but other intermediate distances E can also be used.

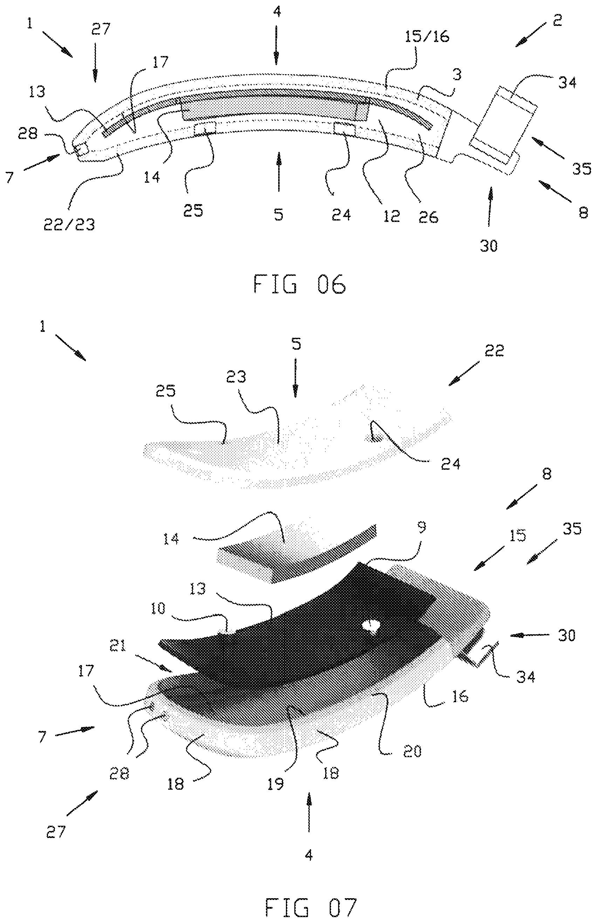

As shown in more detail in figures 6 and 7, the shell-shaped housing 3 of the wearable measuring device 1 encloses an internal space or cavity 12 for housing a printed circuit board (PCB) 13 and a battery 14.

In this case, the first part 15 of the casing 3, i.e. the part 15 that contains the convex side 4, forms a cock-shaped element 16.

The convex side 4 of the housing 3 forms the bottom 17 of the tub-shaped element 16 and is surrounded by upright side walls 18.

The edges 19 at the top 20 of these upright side walls 18 enclose an opening 21.

A second part 22 of the housing 3 has the shape of a concave lid 23 that can be fitted into the opening 21 mentioned above.

The sensors 9 and 10 are mounted on the PCB 13, and holes 24 and 25 are provided in the above-mentioned concave lid for the sensors 9 and 1.

After the introduction of the sensors 9 and 10 in these holes 24 and 25, the head of each sensor 9 and 10 will be situated in the plane of the concave side 5, which is clearly shown in figure 2.

In a preferred embodiment, the printed circuit board (PCB) 13, which is provided in the shell-shaped housing 3, is made flexible in such a way that it can be easily adapted to the shell shape of the housing 3.

Another preferred aspect of the invention consists in that the battery 14, just as the housing 3, is shell-shaped, so that this battery 14 can be easily inserted in the internal space or cavity 12 in the housing 3.

In yet another preferred embodiment of a wearable measuring device 1 according to the invention is further provided a resin 26 in the internal space or cavity 12 in the shell-shaped housing 3, at least in the part thereof that is not filled with electronic or electrical components, such as the battery 14 and the PCB 13 or any other elements.

Preferably, according to the invention, two contacts 28 are provided on one free longitudinal end 27 of the housing 3 to charge the battery 14 inside the housing 3.

In the embodiment shown in figures 1 to 7, these electrical contacts 28 are provided on the front side 7 of the housing 3.

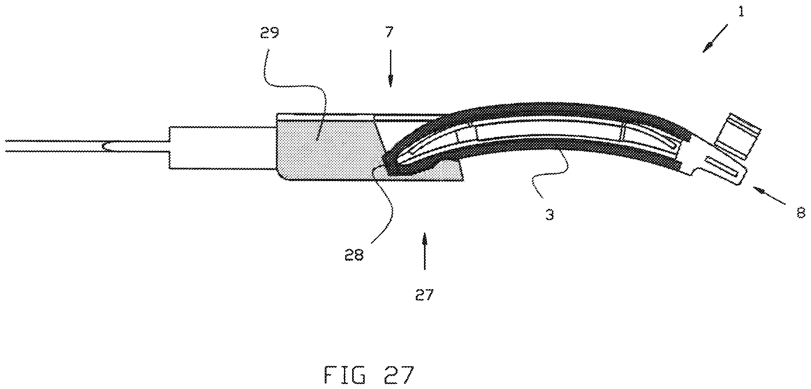

Thus, the measuring device 1 can be charged in a battery charger 29 by resting it with the front side 7 in this battery charger 29, which is illustrated by way of example in figure 27.

However, according to the invention, it is not excluded for electrical contacts to be provided in other places and for the battery charger 29 to be modified accordingly.

Another very important aspect of the invention is that the wearable measuring device 1 or measuring block 2 is intended to be attached to the user's garment or to an accessory worn by the user, such as a watch, bra or belt or the like.

For this purpose, the wearable measuring device 1 is equipped with fixing means 30.

In the embodiment of figures 1 to 8 included, the wearable measuring device 1 is intended to be attached to the wristband 31 of a watch 32 worn around the user's wrist 33.

In this case, the above-mentioned fixing means 30 are carried out as a hook or clips 34 that can be hooked behind or around a part of the garment or accessory in question, in this case behind the watch strap 31.

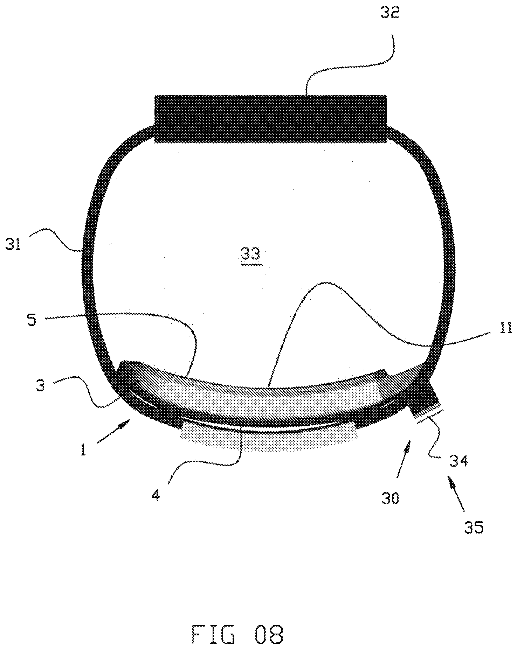

This is illustrated in figure 8.

The hook or clips 34 extend upwards in this case in a direction away from the convex side 4.

It is clear that in this case the fixing means 30 are such that the wearable measuring device 1, after it has been applied, is situated between the watch strap 31 and the person's skin 11, with the electronic sensors 9 and 10 on the concave side 5 being pressed or held against the skin 11.

A major advantage of this embodiment is that the wearable measuring device 1 is very discreetly positioned while wearing it and is hidden between a watch strap 31 and the skin 11.

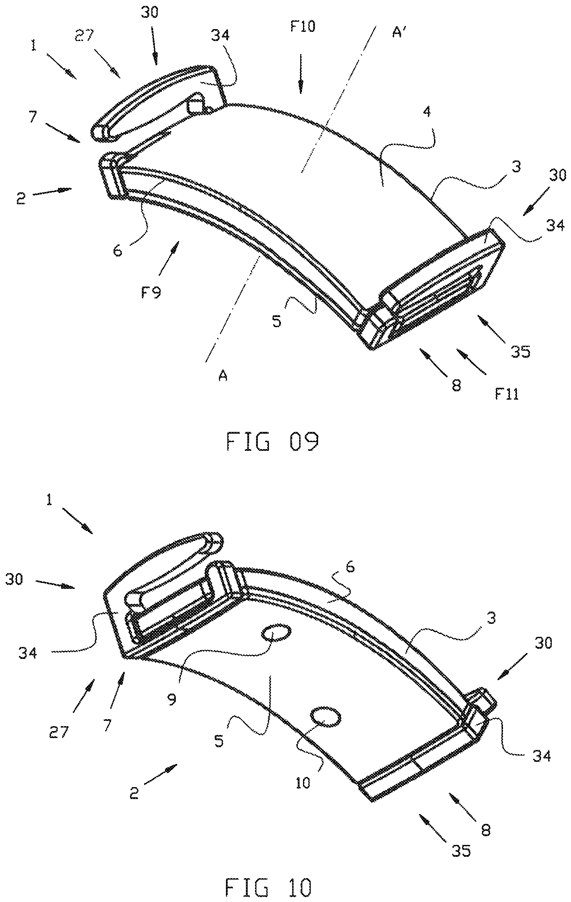

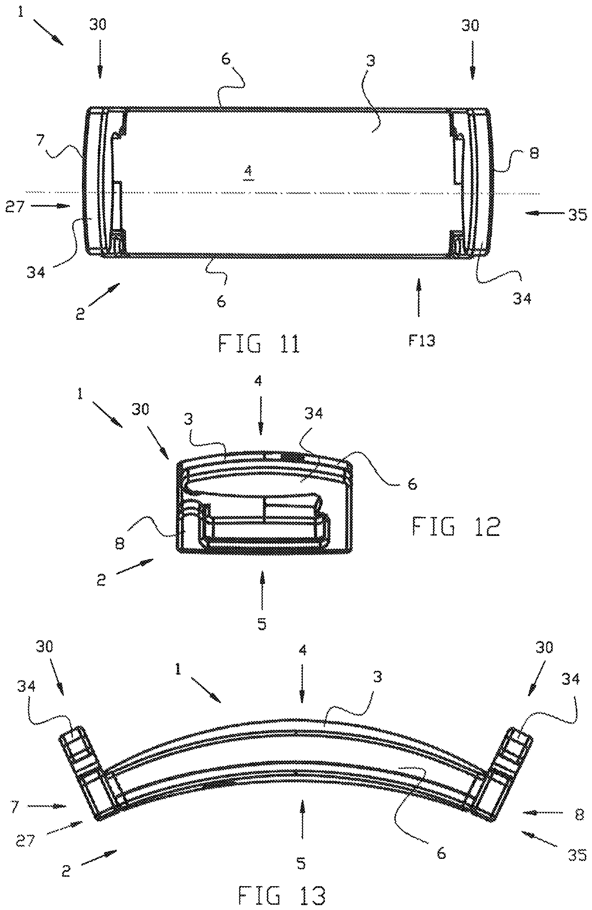

Figures 9 to 13 show another possible embodiment of a wearable measuring device 1 according to the invention.

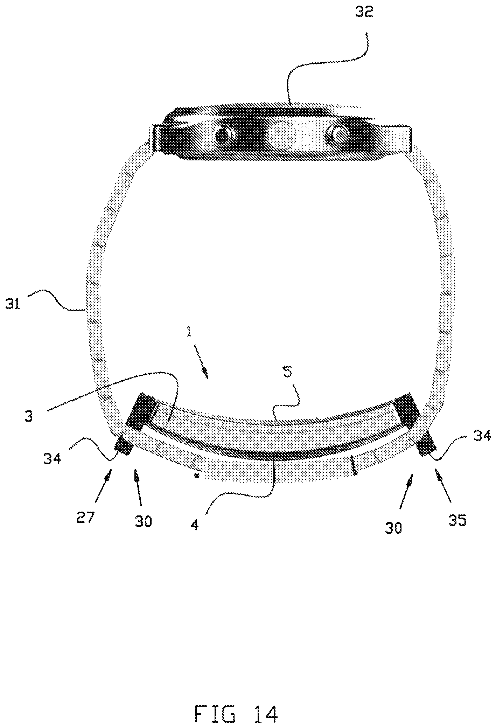

Just as in the previous embodiment, this wearable measuring device 1 is intended to be attached to the watch strap 31 of a 32 watch, wherein the wearable measuring device 1 is also placed between the watch strap 31 and the skin 11, which is illustrated in figure 14.

The difference with the previous embodiment is that in this case fixing means 30 are provided at both free ends 27 and 35 of the housing 3, whereas in the previous embodiment of figures 1 to 7 included the fixing means 30 are provided only at one free longitudinal end 35 of the housing 3.

The fixing means 30 are again formed by hooks or clips 34, both of which, as in the previous case, extend upwards in a direction away from the convex side 4 of the housing 3.

The front side 7 and the back side 8 of the wearable measuring device 1 are both identically configured in the embodiment of figures 9 to 13 included, and in particular both are of a linear or substantially linear design.

Also, in this embodiment there is no longer really a front 7 side and a back 8 side, since the wearable measuring device 1 is symmetrical, so that at a rotation of 180° around an axis AA' perpendicular to the convex side 4 through its centre, exactly the same situation is obtained.

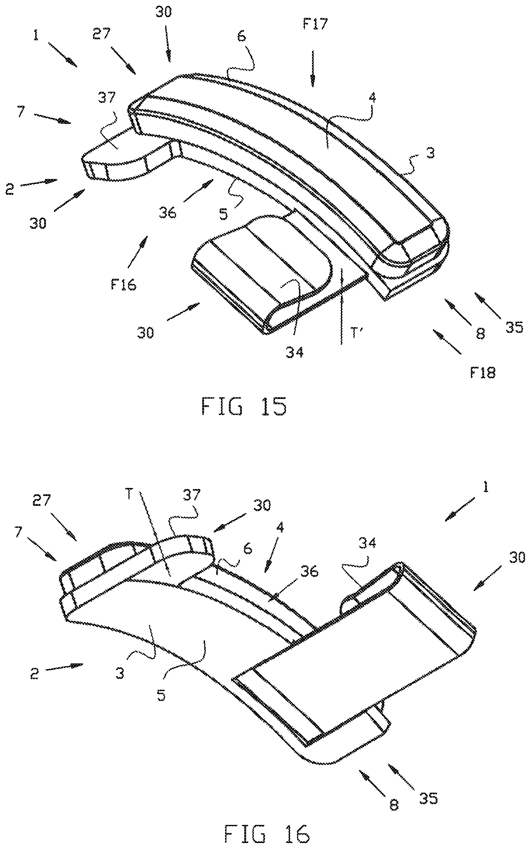

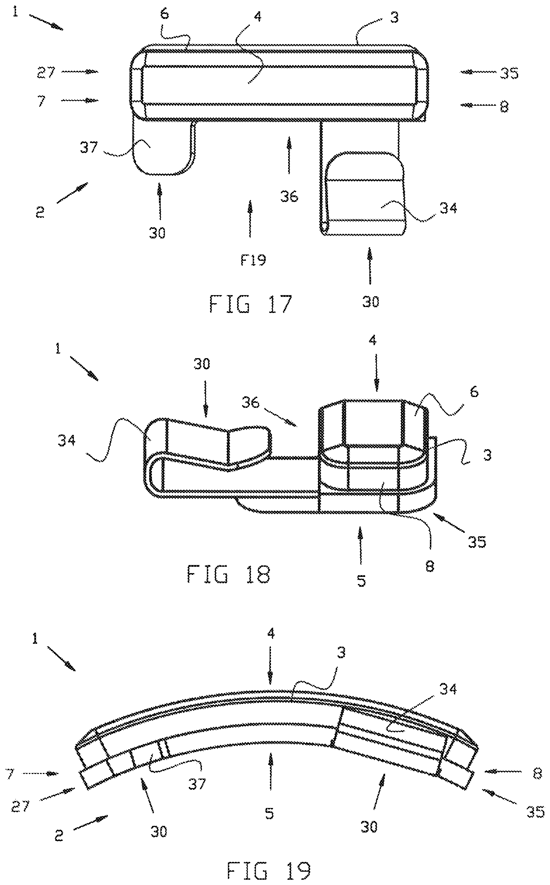

Figures 15 to 19 show a third possible embodiment of a wearable measuring device 1 according to the invention, which is again intended to be attached to the strap 31 of a watch 32.

In this embodiment are again provided fixing means 30 to this end at both the free ends 27 and 35 of the housing 3.

However, in this case, these fixing means 30 extend sideways from the housing 3, in particular sideways from one longitudinal side 36 of this housing 3 at the level of the base 17 or the concave side 5 of the housing 3.

At the free end 35, the fixing means 30 are again formed of a kind of hook or clips 34 intended to partially encase a watch strap 31.

At the other free end 27, the fixing means 30 are formed solely by a flat, laterally extending protuberance 37, which is intended to be inserted between the watch strap 31 and the skin 11 of the user in order to hold the wearable measuring device 1 correctly against the skin 11.

It is clear that in this embodiment, the wearable measuring device 1, after attaching it to a watch strap 31, is located next to this watch strap 31 and is being held against the skin 11 there by the fixing means 30.

A major advantage of this embodiment is that, when worn, the parts of the wearable measuring device 1 located between the watch strap 31 and the skin 1, in particular a part of the hook 34 and the flat protrusion 37, are not or hardly noticeable to the user due to their small thicknesses T and T'.

However, this embodiment can only be worn in a slightly less discrete manner than in the previous embodiments.

Indeed, in the previous cases, the wearable measuring device 1 is situated entirely between the watch strap 31 and the skin 11 while wearing it, whereas in the case of figures 15 to 19 included, the wearable measuring device 1 is situated next to the watch strap 31 while wearing it.

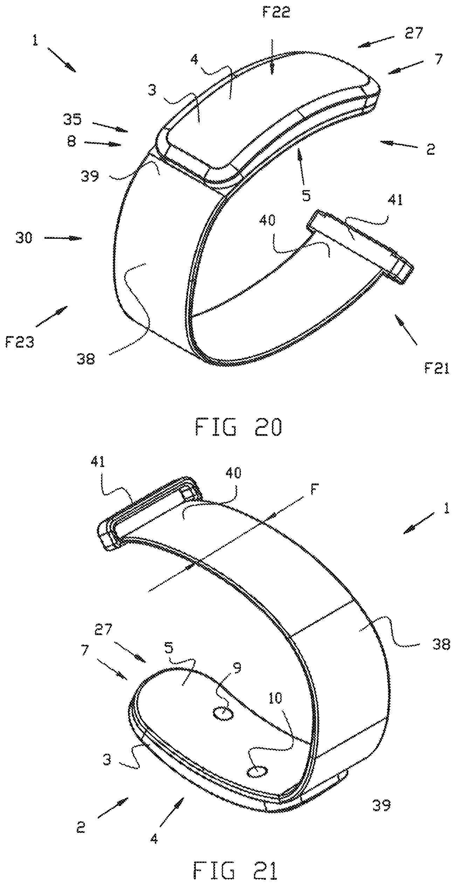

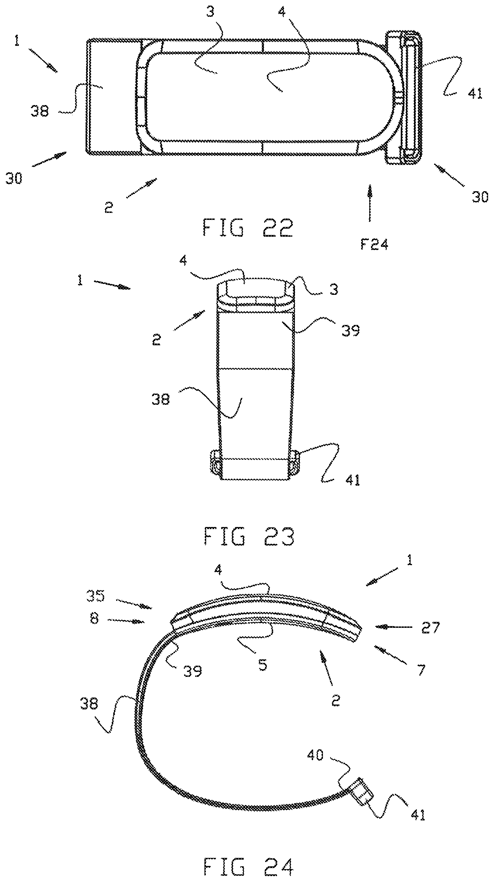

Figures 20 to 24 show a fourth possible embodiment of a wearable measuring device 1 according to the invention, which is again intended to be attached to the strap 31 of a watch 32.

In this case, however, the fixing means 30 are formed by a flexible strap 38.

This flexible strap 38 is securely attached at one end 39 to the housing 3 of the measuring device 1, in particular at the free end 35 of the housing 3, and the flexible strap 38 extends in line with the length of this elongated housing 3.

At its other free end 40, the flexible strap 38 is equipped with a ring-shaped element 41 to enclose part of the garment or accessory to which the wearable measuring device 1 is to be attached.

In this case, this ring-shaped element 41 is intended to enclose a watch strap 31, in particular in a spot at a certain distance from the housing 3, in accordance with the length of the flexible strap 38.

The flexible strap has a width F corresponding to the width B of the housing 3 as well as to the width of the watch strap 31.

It is clear that this embodiment of a wearable measuring device 1 according to the invention can be worn again in a more discrete manner, just as in the first two embodiments discussed, wherein the wearable measuring device 1 is situated between a watch strap 31 and the skin 1 while wearing it.

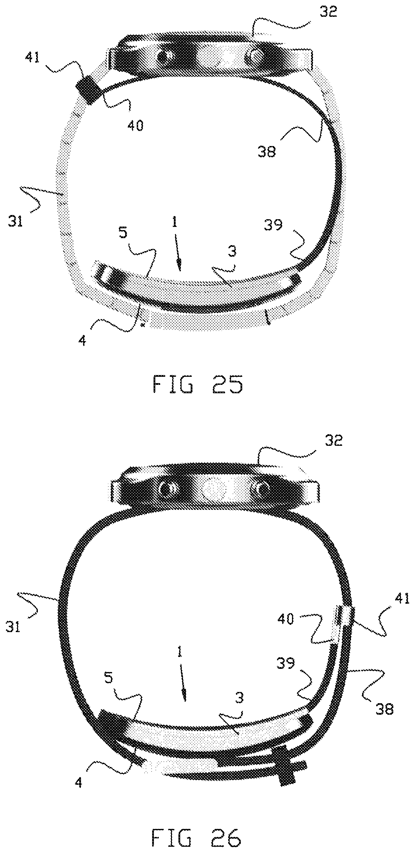

This is illustrated in figure 25.

Figure 26 shows the use of a wearable measuring device 1 according to the invention in a slightly modified embodiment, wherein this time the length of the flexible strap 38 has been shortened compared to the previous embodiment shown in figures 20 to 25 included.

In particular, the length of the flexible strap 38 in the previous embodiment is substantially larger than half the length of the watch strap 31, whereas in the illustration of figure 26 this length is substantially shorter than half the length of the watch strap 31.

In a special embodiment of a wearable measuring device 1 according to the invention, which is not further shown in the figures, the fixing means 30 comprise closing means for closing and re-opening the fixing means 30, respectively during the attachment and the release of the fixing means.

Another interesting aspect of the invention is that a central part of the shell-shaped housing 3 is made of a rigid material that is little or not deformable and at least one of the ends 27 and/or 35 is made of a more deformable and flexible material that allows the housing 3 to be deformed at the relevant ends 27 and/or 35 in order to adapt more easily to local restrictions.

The central part of the shell-shaped housing 3 could be made for example of a rigid plastic and the more deformable and flexible material of the above-mentioned end 27 or 35 or the abovementioned ends 27 and 35 could be, for example, a silicone.

Preferably, fixing means 30 should be provided at one end 27 or 35 which is made of a flexible material and/or which is flexibly connected to the more rigid central section of the shell-shaped housing 3.

The result is that the orientation and/or positioning of the more rigid central part of the shell-shaped housing 3 experiences more freedom. In other words, wearing the wearable measuring device 1 becomes more pleasant.

The invention is by no means limited to the embodiments of a wearable measuring device 1 according to the invention described by way of example and illustrated by the figures; on the contrary, such a wearable measuring device 1 can be achieved in other ways while still remaining within the scope of the invention.

## Claims

1. Wearable measuring device (1) which can be worn on a person's body and which is intended to measure parameters on the person's skin (11), whereby the measuring device (1) is made as a measuring block (2) provided with an elongated, slightly curved housing (3), in particular as if it were a segment of a cylinder housing with a radius of between 52 and 62 millimetres, or rather between 54 and 60 millimetres, and preferably between 56 and 58 millimetres, and with a convex side (4) and a concave side (5) and with the surface of the concave side (5) being equipped with at least two electronic sensors (9, 10) intended to be put in contact with the skin (11) on the inside of the wrist of the person concerned for measuring the parameters, wherein the wearable measuring device (1) does not have a wristband or the like, whereby the wearable measuring block is suitable to be located between a wristband, for example of a usual wristwatch, and wrist, whereby the measuring block is suitable to be used between the wristband and the skin on the inside of the wrist and is provided to this end with fixing means, wherein the fixing means contain a hook or clips which can be hooked behind or around a part of a said wristband, whereby the measuring block (2) contains at least two electronic sensors (9, 10) for measuring the activity of the sweat glands in the area concerned of the skin (11), also known as 'galvanic skin response', wherein at least two sensors are provided at a mutual distance of at least 20 millimetres and wherein the sensors have a rounded contact surface.

2. Wearable measuring device (1) according to claim1, **characterised in that** the fixing means (30) contain one or several of the following elements:
- a flexible strap (38) fixed at one end (39) to the housing (3) and provided at the other free end (40) with a ring-shaped element (41) with which a part of said garment or accessory (31, 32) can be enclosed;
- closing means for closing and re-opening the fixing means (30), respectively during the attachment and re-opening of the fixing means (30).

3. Wearable measuring device (1) according to claim 1 or 2, **characterised in that** the fixing means (30) are provided at one free longitudinal end (27, 35) of the housing.

4. Wearable measuring device (1) according to claim 1 or 2, **characterised in that** fixing means (30) are provided at both free ends (27, 35) of the housing (3).

5. Wearable measuring device (1) according to one or several of the preceding claims, **characterised in that** at one free longitudinal end (27) of the housing (3) are provided two electrical contacts (28) for charging a battery (14) inside the housing (3), with the recesses in the housing having rounded edges.

6. Wearable measuring device (1) according to one or several of the preceding claims, **characterised in that** the shell-shaped housing (3) has a constant width (B) and a constant thickness (D) over most of its length (L).

7. Wearable measuring device (1) according to one or several of the preceding claims, **characterised in that** the shell-shaped housing encloses an internal space or cavity (12) for housing a printed circuit board (PCB) (13) and a battery (14), the first part (15) of the housing (3) containing the convex side (4) forming a tub-shaped element (16) whose edges (19) enclose an opening (21) and the second part (22) of the housing (3) having the shape of a concave lid (23), which can be fitted into the above-mentioned opening (21).

8. Wearable measuring device (1) according to one or several of the preceding claims, **characterised in that** a printed circuit board (PCB) (13) is provided in the shell-shaped housing (3), which has a flexible design.

9. Wearable measuring device (1) according to one or several of the preceding claims, **characterised in that** the shell-shaped housing (3) contains a battery (14) that is also designed as a shell.

10. Wearable measuring device (1) according to one or several of the preceding claims, **characterised in that** for a watertight or splash-proof embodiment, a resin (26) is incorporated in the internal space or cavity 12 of the shell-shaped housing (3), at least in the part which is not filled with electronic components (13, 14).

11. Wearable measuring device (1) according to one or several of the preceding claims, **characterised in that** the central part of the shell-shaped housing (3) is made of a rigid material which has little or no deformability and wherein at least one of the ends (27, 35) provided and hidden under the wristband while in use, is made of a more deformable and flexible material and/or is connected in a flexible way to the more rigid central part of the shell-shaped housing (3), thus allowing the housing (3) to be deformed at the ends (27, 35) concerned in order to adapt more easily there to local restrictions.

12. Wearable measuring device (1) according to claim 11, **characterised in that** the fixing means (30) are provided at one end (27, 35) made of a flexible material and/or is flexibly connected to the more rigid central section of the shell-shaped housing (3).

13. Wearable measuring device (1) according to one or several of the preceding claims, **characterised in that** it is equipped with transmitting devices to wirelessly transmit data to an output device, such as a mobile phone, PC, laptop or the like.

14. Combination of a wearable measuring device (1) according to claim 13 and an output device, **characterised in that** the output device is provided with receiving means with which data from the measuring device (1) can be received wirelessly, and with an app or software application allowing these data to be processed and presented to a user in a processed form.

## Patentansprüche

1. Tragbares Messgerät (1), das an dem Körper einer Person getragen werden kann und das zur Messung von Parametern auf der Haut (11) der Person bestimmt ist, wobei das Messgerät (1) als Messblock (2) ausgebildet ist, der mit einem länglichen, leicht gekrümmten Gehäuse (3), insbesondere als ob es sich um ein Segment eines Zylindergehäuses mit einem Radius zwischen 52 und 62 Millimetern, vorzugsweise zwischen 54 und 60 Millimetern, und vorzugsweise zwischen 56 und 58 Millimetern handeln würde, und mit einer konvexen Seite (4) und einer konkaven Seite (5), versehen ist, und wobei die Oberfläche der konkaven Seite (5) mit mindestens zwei elektronischen Sensoren (9, 10) ausgestattet ist, die dazu bestimmt sind, mit der Haut (11) auf der inneren Seite des Handgelenks der betreffenden Person in Kontakt gebracht zu werden, um die Parameter zu messen, wobei das tragbare Messgerät (1) kein Armband oder dergleichen aufweist, wobei der tragbare Messblock geeignet ist, zwischen einem Armband, beispielsweise einer üblichen Armbanduhr, und dem Handgelenk angeordnet zu werden, wobei der Messblock geeignet ist, zwischen dem Armband und der Haut auf der inneren Seite des Handgelenks verwendet zu werden, und zu diesem Zweck mit Befestigungsmitteln versehen ist, wobei die Befestigungsmittel einen Haken oder Clips enthalten, die hinter oder um einen Teil des genannten Armbands gehakt werden können, wobei der Messblock (2) mindestens zwei elektronische Sensoren (9, 10) zur Messung der Aktivität der Schweißdrüsen in dem betreffenden Bereich der Haut (11), auch bekannt als "galvanische Hautreaktion", enthält, wobei mindestens zwei Sensoren in einem gegenseitigen Abstand von mindestens 20 Millimetern vorgesehen sind und wobei die Sensoren eine abgerundete Kontaktfläche aufweisen.

2. Tragbares Messgerät (1) nach Anspruch 1, **dadurch gekennzeichnet, dass** die Befestigungsmittel (30) eines oder mehrere der folgenden Elemente enthalten:
- ein flexibles Band (38), das an einem Ende (39) an dem Gehäuse (3) befestigt und an dem anderen freien Ende (40) mit einem ringförmigen Element (41) versehen ist, mit dem ein Teil des Kleidungsstücks oder Zubehörs (31, 32) umschlossen werden kann;
- Schließmittel zum Schließen und Wiederöffnen der Befestigungsmittel (30) während der Befestigung bzw. des Wiederöffnens der Befestigungsmittel (30).

3. Tragbares Messgerät (1) nach Anspruch 1 oder 2, **dadurch gekennzeichnet, dass** die Befestigungsmittel (30) an einem freien Längsende (27, 35) des Gehäuses vorgesehen sind.

4. Tragbares Messgerät (1) nach Anspruch 1 oder 2, **dadurch gekennzeichnet, dass** an beiden freien Enden (27, 35) des Gehäuses (3) Befestigungsmittel (30) vorgesehen sind.

5. Tragbares Messgerät (1) nach einem oder mehreren der vorhergehenden Ansprüche, **dadurch gekennzeichnet, dass** an einem freien Längsende (27) des Gehäuses (3) zwei elektrische Kontakte (28) zum Laden einer Batterie (14) im Inneren des Gehäuses (3) vorgesehen sind, wobei die Ausnehmungen in dem Gehäuse abgerundete Kanten aufweisen.

6. Tragbares Messgerät (1) nach einem oder mehreren der vorhergehenden Ansprüche, **dadurch gekennzeichnet, dass** das schalenförmige Gehäuse (3) über den größten Teil seiner Länge (L) eine konstante Breite (B) und eine konstante Dicke (D) aufweist.

7. Tragbares Messgerät (1) nach einem oder mehreren der vorhergehenden Ansprüche, **dadurch gekennzeichnet, dass** das schalenförmige Gehäuse einen Innenraum bzw. Hohlraum (12) zur Aufnahme einer Leiterplatte (PCB) (13) und einer Batterie (14) umschließt, wobei der erste Teil (15) des Gehäuses (3), der die konvexe Seite (4) enthält, ein wannenförmiges Element (16) bildet, dessen Ränder (19) eine Öffnung (21) einschließen, und wobei der zweite Teil (22) des Gehäuses (3) die Form eines konkaven Deckels (23) hat, der in die oben genannte Öffnung (21) eingesetzt werden kann.

8. Tragbares Messgerät (1) nach einem oder mehreren der vorhergehenden Ansprüche, **dadurch gekennzeichnet, dass** in dem schalenförmigen Gehäuse (3), das flexibel ausgebildet ist, eine Leiterplatte (PCB) (13) vorgesehen ist.

9. Tragbares Messgerät (1) nach einem oder mehreren der vorhergehenden Ansprüche, **dadurch gekennzeichnet, dass** das schalenförmige Gehäuse (3) eine Batterie (14) enthält, die ebenfalls in der Form einer Schale ausgebildet ist.

10. Tragbares Messgerät (1) nach einem oder mehreren der vorhergehenden Ansprüche, **dadurch gekennzeichnet, dass** für eine wasserdichte oder spritzwassergeschützte Ausführung in den Innenraum oder Hohlraum (12) des schalenförmigen Gehäuses (3) zumindest in dem Teil, der nicht mit elektronischen Bauteilen (13, 14) gefüllt ist, ein Harz (26) eingebracht ist.

11. Tragbares Messgerät (1) nach einem oder mehreren der vorhergehenden Ansprüche, **dadurch gekennzeichnet, dass** der zentrale Teil des schalenförmigen Gehäuses (3) aus einem starren Material besteht, das wenig oder gar nicht verformbar ist, und wobei mindestens eines der Enden (27, 35), das während des Gebrauchs unter dem Armband vorgesehen und verborgen ist, aus einem verformbareren und flexibleren Material besteht und/oder auf flexible Weise mit dem steiferen Mittelteil des schalenförmigen Gehäuses (3) verbunden ist, wodurch das Gehäuse (3) an den betreffenden Enden (27, 35) verformt werden kann, um sich dort leichter an örtliche Einschränkungen anzupassen.

12. Tragbares Messgerät (1) nach Anspruch 11, **dadurch gekennzeichnet, dass** die Befestigungsmittel (30) an einem Ende (27, 35) vorgesehen sind, das aus einem flexiblen Material hergestellt wird, und/oder das mit dem steiferen Mittelteil des schalenförmigen Gehäuses (3) flexibel verbunden ist.

13. Tragbares Messgerät (1) nach einem oder mehreren der vorhergehenden Ansprüche, **dadurch gekennzeichnet, dass** es mit Sendeeinrichtungen zur drahtlosen Übertragung von Daten an ein Ausgabegerät, wie z.B. ein Mobiltelefon, PC, Laptop oder dergleichen, ausgestattet ist.

14. Kombination aus einem tragbaren Messgerät (1) nach Anspruch 13 und einem Ausgabegerät, **dadurch gekennzeichnet, dass** das Ausgabegerät mit Empfangsmitteln versehen ist, mit denen Daten von dem Messgerät (1) drahtlos empfangen werden können, und mit einer App oder Softwareanwendung, die es erlaubt, diese Daten zu verarbeiten und einem Benutzer in verarbeiteter Form zu präsentieren.

## Revendications

1. Dispositif de mesure portatif (1) qui peut être porté sur le corps d'une personne et qui est destiné à mesurer des paramètres sur la peau (11) de la personne, dans lequel le dispositif de mesure (1) est réalisé sous la forme d'un bloc de mesure (2) qui est équipé d'un boîtier allongé (3) de forme légèrement courbe, en particulier en quelque sorte sous la forme d'un segment d'un boîtier cylindrique qui possède un rayon situé entre 52 et 62 millimètres, ou plutôt entre 54 et 60 millimètres, et de préférence entre 56 et 58 millimètres, et qui possède un côté convexe (4) et un côté concave (5) et dans lequel la surface du côté concave (5) est équipée d'au moins deux capteurs électroniques (9, 10) qui sont destinés à être mis en contact avec la peau (11) sur la face interne du poignet de la personne concernée pour la mesure des paramètres, dans lequel le dispositif de mesure portatif (1) est dépourvu d'un bracelet ou analogue, dans lequel le bloc de mesure portatif est approprié pour être disposé entre un bracelet, par exemple d'une montre-bracelet habituelle, et le poignet, dans lequel le bloc de mesure est approprié pour être utilisé entre le bracelet et la peau sur la face interne du poignet et est équipé à cet effet de moyens de fixation, dans lequel les moyens de fixation contiennent un crochet ou des pinces qui peut/peuvent venir s'accrocher derrière ou autour d'une partie du bracelet en question, dans lequel le bloc de mesure (2) contient au moins deux capteurs électroniques (9, 10) destinés à mesurer l'activité des glandes sudoripares dans la zone concernée de la peau (11), ce que l'on désigne également par l'expression « réponse galvanique cutanée », dans lequel au moins deux capteurs sont prévus à une distance réciproque d'au moins 20 millimètres, et dans lequel les capteurs possèdent une surface de contact de forme arrondie.

2. Dispositif de mesure portatif (1) selon la revendication 1, **caractérisé en ce que** les moyens de fixation (30) contiennent un ou plusieurs éléments choisis parmi les éléments indiqués ci-après, à savoir :
- une bande flexible (38) qui est fixée, à une extrémité (39), au boîtier, et qui est munie, à l'autre extrémité libre (40), d'un élément (41) possédant une configuration de forme annulaire, avec lequel une partie dudit vêtement ou dudit accessoire (31, 32) peut être enfermée ;
- des moyens de fermeture destinés à fermer et réouvrir les moyens de fixation (30), respectivement au cours de l'accrochage et de la réouverture des moyens de fixation (30).

3. Dispositif de mesure portatif (1) selon la revendication 1 ou 2, **caractérisé en ce que** les moyens de fixation (30) sont prévus à une extrémité longitudinale libre (27, 35) du boîtier.

4. Dispositif de mesure portatif (1) selon la revendication 1 ou 2, **caractérisé en ce que** les moyens de fixation (30) sont prévus aux deux extrémités libres (27, 35) du boîtier (3).

5. Dispositif de mesure portatif (1) selon une ou plusieurs des revendications précédentes, **caractérisé en ce que**, à une extrémité longitudinale libre (27) du boîtier (3), on prévoit deux contacts électriques (28) pour recharger un accumulateur (14) à l'intérieur du boîtier (3), les évidements dans le boîtier possédant des bords de forme arrondie.

6. Dispositif de mesure portatif (1) selon une ou plusieurs des revendications précédentes, **caractérisé en ce que** le boîtier (3) avec une configuration en forme de coquille possède une largeur constante (B) et une épaisseur constante (D) sur la majeure partie de sa longueur (L).

7. Dispositif de mesure portatif (1) selon une ou plusieurs des revendications précédentes, **caractérisé en ce que** le boîtier possédant une configuration en forme de coquille renferme une cavité ou un espace interne (12) pour que viennent s'y loger une carte de circuits imprimés (PCB) (13) et un accumulateur (14), la première partie (15) du boîtier (3) contenant le côté convexe (4) qui forme un élément (16) possédant une configuration de forme tubulaire dont les bords (19) renferment une ouverture (21) et la deuxième partie (22) du boîtier (3) possédant la configuration d'un couvercle concave (23) qui peut venir s'insérer dans l'ouverture (21) qui a été mentionnée ci-dessus.

8. Dispositif de mesure portatif (1) selon une ou plusieurs des revendications précédentes, **caractérisé en ce que** l'on prévoit une carte de circuits imprimés (PCB) (13) dans le boîtier (3) possédant une configuration en forme de coquille, qui est de conception flexible.

9. Dispositif de mesure portatif (1) selon une ou plusieurs des revendications précédentes, **caractérisé en ce que** le boîtier (3) possédant une configuration en forme de coquille contient un accumulateur (14) qui est également conçu sous la forme d'une coquille.

10. Dispositif de mesure portatif (1) selon une ou plusieurs des revendications précédentes, **caractérisé en ce que**, pour une forme de réalisation qui procure une étanchéité à l'eau ou une résistance aux éclaboussures, une résine (26) est incorporée dans la cavité ou l'espace interne (12) du boîtier (3) possédant une configuration en forme de coquille, dont au moins une partie n'est pas remplie avec des composants électroniques (13, 14).

11. Dispositif de mesure portatif (1) selon une ou plusieurs des revendications précédentes, **caractérisé en ce que** la partie centrale du boîtier (3) possédant une configuration en forme de coquille est réalisée à partir d'un matériau rigide qui possède une aptitude à la déformation minime voire nulle, et dans lequel au moins une des extrémités (27, 35) qui a été prévue et qui est cachée en dessous du bracelet lors de l'utilisation, est réalisée à partir d'un matériau plus déformable et flexible et/ou est reliée d'une manière flexible à la partie centrale plus rigide du boîtier (3) possédant une configuration en forme de coquille, ce qui permet une déformation du boîtier (3) aux extrémités concernées (27, 35), dans le but d'adapter plus aisément cet endroit à des restrictions locales.

12. Dispositif de mesure portatif (1) selon la revendication 11, **caractérisé en ce que** les moyens de fixation (30) sont prévus à une extrémité (27, 35) qui est réalisée à partir d'un matériau flexible et/ou qui est reliée de manière flexible au tronçon central plus rigide du boîtier (3) possédant une configuration en forme de coquille.

13. Dispositif de mesure portatif (1) selon une ou plusieurs des revendications précédentes, **caractérisé en ce qu'**il est équipé de dispositifs de transmission pour transmettre sans fil des données à un dispositif de sortie, tel qu'un téléphone portable, un PC, un ordinateur portable ou analogue.

14. Combinaison d'un dispositif de mesure portatif (1) selon la revendication 13 et d'un dispositif de sortie, **caractérisée en ce que** le dispositif de sortie est équipé de moyens de réception avec lesquels des données émises par le dispositif de mesure (1) peuvent être reçues sans fil, et d'une application ou d'un logiciel qui permet de traiter lesdites données et de les présenter à un utilisateur sous une forme qui a été traitée.
